# EUROPÄISCHE PATENTSCHRIFT

(11) **EP 1 636 158 B1**
(45) Veröffentlichungstag und Bekanntmachung des Hinweises auf die Patenterteilung: **10.10.2007**
(21) Anmeldenummer: 04739689.0
(22) Anmeldetag: 08.06.2004
(51) Int. Cl.: C07C 43/16, C07C 41/42, C07C 41/08, C07C 29/82

(54) **VERFAHREN ZUR DESTILLATIVEN TRENNUNG EINES VINYLETHER UND ALKOHOL ENTHALTENDEN GEMISCHS**
METHOD FOR THE DISTILLATIVE SEPARATION OF A MIXTURE CONTAINING VINYL ETHER AND ALCOHOL
PROCEDE DE SEPARATION PAR DISTILLATION D'UN MELANGE CONTENANT DE L'ETHER VINYLIQUE ET DE L'ALCOOL

(30) Priorität: 12.06.2003 DE 10326403
(43) Veröffentlichungstag der Anmeldung: 22.03.2006
(73) Patentinhaber: BASF Aktiengesellschaft, 67056 Ludwigshafen (DE)
(72) Erfinder: KLASS, Katrin, 68159 Mannheim (DE); BECKER, Heike, 68163 Mannheim (DE); VOGELSANG, Regina, 67061 Ludwigshafen (DE); HAUK, Alexander, 67071 Ludwigshafen (DE); SIEGERT, Markus, 69126 Heidelberg (DE); HENKELMANN, Jochem, 68165 Mannheim (DE)
(86) Internationale Anmeldenummer: PCT/EP2004/006160
(87) Internationale Veröffentlichungsnummer: WO 2004/110970

(56) Entgegenhaltungen:
- WO-A-00/15590
- US-A- 3 878 058
- DATABASE WPI Section Ch, Week 9827 Derwent Publications Ltd., London, GB; Class E15, AN 1998-306099 XP002298960 & JP 10 109952 A (MARUZEN PETROCHEM) 28. April 1998 (1998-04-28)
- DATABASE WPI Section Ch, Week 9148 Derwent Publications Ltd., London, GB; Class E17, AN 1991-351559 XP002298961 & SU 1 616 888 A (BABAKOVA) 30. Dezember 1990 (1990-12-30) in der Anmeldung erwähnt

## Beschreibung

Die vorliegende Erfindung betrifft ein Verfahren zur destillativen Trennung eines Vinylether der allgemeinen Formel (I)

R¹-O-CH=CH₂ (I),

und Alkohol der allgemeinen Formel (II)

R²-OH (II),

in der R¹ und R² unabhängig voneinander einen C₂-bis C₄-Alkylrest bedeuten, enthaltenden Gemischs, bei dem der Alkohol (II) einen um mindestens 1°C höheren Siedepunkt, gemessen bei oder extrapoliert auf 0,1 MPa abs, als der Vinylether (II) aufweist.

Vinylether stellen eine wichtige Verbindungsklasse mit breitem Einsatzgebiet dar. So finden sie unter anderem Verwendung als Monomerbausteine in Polymeren und Copolymeren, in Beschichtungen, Klebstoffen, Druckfarben sowie in strahlungshärtenden Lacken. Weitere Anwendungsgebiete sind die Herstellung von Zwischenprodukten, Geruchs- und Geschmacksstoffen sowie pharmazeutischen Produkten.

Die technische Herstellung von Vinylethern erfolgt in der Regel durch Umsetzung der entsprechenden Alkohole mit Ethin in Gegenwart basischer Katalysatoren (siehe Ullmann's Encyclopedia of Industrial Chemistry, 6th edition, 2000 Electronic Release, Chapter "VINYL ETHERS - Production" und W. Reppe et al., Justus Liebigs Ann. Chem., 601 (1956), Seiten 135 bis 138). Das dabei gebildete Reaktionsgemisch enthält neben dem Katalysator und möglichen Nebenprodukten hauptsächlich den gebildeten Vinylether und den nicht-umgesetzten Alkohol. Die beiden letztgenannten Komponenten sind bis auf die C₁-Derivate Methylvinylether/Methanol aufgrund Azeotropbildung nicht durch einfache Destillation in Fraktionen der gewünschten Reinheit trennbar. Dieses Problem wurde bisher durch den Einsatz extraktiver und extrativdestillativer Verfahren gelöst, bei denen man eine oder mehrere Fremdsubstanzen als Hilfsmittel zufügt und diese nach Auftrennung in eine Vinylether- und eine Alkohol-enthaltende Fraktion wieder abtrennt.

So beschreibt EP-A 0 415 334 die ein- oder mehrstufige Extraktion eines Vinylether und Alkohol enthaltenden Gemischs mit einer wässrigen Lösung einer Base, bei der eine Vinylether-Phase und eine wässrige alkoholische Phase erhalten und der Alkohol anschließend aus der wässrigen alkoholischen Phase destillativ gewonnen wird.

US 2,779,720 beschreibt ein Verfahren zur Trennung eines Gemischs, welches einen aliphatischen Vinylether und einen aliphatischen Alkohol enthält, bei dem dieses Gemisch zusammen mit Wasser und einem Glykol oder Glykolether destilliert wird. Als Kopfprodukt bildet sich dabei ein azeotropes Gemisch aus dem Vinylether und Wasser, aus dem in einer weiteren Destillation der Vinylether gewonnen werden kann. Als Sumpfprodukt verbleibt ein den Alkohol und Glykol oder Glykolether enthaltendes Gemisch, aus dem durch eine nachgeschaltete Destillation der Alkohol gewonnen werden kann.

DE-A 1 000 804 offenbart ein Verfahren zur Aufarbeitung eines Gemischs, welches einen Monovinylether eines mehrwertigen Alkohols und einen mehrwertigen Alkohol enthält, bei dem dieses Gemisch im alkalischen Medium zusammen mit Wasser destilliert oder einer Wasserdampfdestillation unterworfen wird. Dabei wird als Kopfprodukt eine azeotrope Mischung des Vinylethers mit Wasser erhalten, wobei als Rückstand im Sumpf der Alkohol zurückbleibt. Die bei der Destillation angefallene Kopffraktion kann gegebenenfalls mit einem nicht wasserlöslichen Lösungsmittel extrahiert werden, wobei der Vinylether in die organische Phase übergeht und aus dieser destillativ enthalten werden kann.

SU 1 616 888 beschreibt die Trennung eines Butanol und Butyl-vinylether enthaltenden Gemischs durch Extraktivdestillation mit Wasser. Aus der als Kopfprodukt gebildeten azeotropen Mischung, welche den Butyl-vinylether und Wasser enthält, wird in einer nachfolgenden Destillation der Butyl-vinylether gewonnen. Das Butanol enthaltende Sumpfprodukt der ersten Destillation wird in einer weiteren Destillation aufbereitet und Butanol gewonnen.

US 3,878,058 offenbart ein Verfahren zur Gewinnung von Alkylvinylethern aus einem den Alkylvinylether und einen aliphatischen Alkohol enthaltenden Gemisch, bei dem dieses Gemisch zusammen mit einem Glykol-monoether destilliert wird. Dabei wird als Kopfprodukt der Alkylvinylether abgetrennt und als Sumpfprodukt ein Alkohol und Glykol-monoether enthaltendes Gemisch erhalten. Dieses wird in einer nachgeschalteten Destillation in den Alkohol und den Glykol-monoether getrennt und letzterer zur ersten Destillationsstufe zurückgeführt.

Nachteilig an allen oben genannten Verfahren ist der Zusatz einer oder mehrerer Fremdsubstanzen als Hilfsmittel. Dadurch werden dem System neue Verbindungen hinzugefügt, welche anschließend wieder abgetrennt werden müssen. Damit verbunden ist zum Einen ein entsprechender apparativer und verfahrenstechnischer Aufwand und zum Anderen die Gefahr der Verunreinigung des Vinylethers und/oder des Alkohols durch restliche Mengen dieser Fremdsubstanzen. Des Weiteren verringern die Fremdsubstanzen aufgrund ihres Eigenvolumens die Kapazität der Destillatiansvorrichtungen beziehungsweise bedingen den Einsatz größerer Destillationsvorrichtungen.

Weiterhin offenbart JP 10 109952 ein Verfahren zur Trennung eines Cyclohexylvinylether und Cyclohexanol enthaltenden Gemisches durch Destillation, wobei bei dieser speziellen Destillation eine erste Kolonne in einem Druckbereich von 5 bis 100 mm Hg arbeitet und eine zweite Kolonne in einem Bereich von 150 bis 760 mm Hg.

Demgemäss bestand die Aufgabe, ein Verfahren zur Trennung eines Vinylether und Alkohol enthaltenden Gemischs zu finden, welches die oben genannten Nachteile nicht besitzt und insbesondere mit geringem apparativen und verfahrenstechnischen Aufwand, mit hoher Anlagen-Kapazität und ohne die Gefahr einer Verunreinigung des Vinylethers und/oder des Alkohols durch die Zugabe von Fremdsubstanzen als Hilfsmittel zu aufgereinigtem Vinylether und Alkohol führt.

Demgemäss wurde ein Verfahren zur destillativen Trennung eines Vinylether der allgemeinen Formel (I)

R¹-O-CH=CH₂ (I),

und Alkohol der allgemeinen Formel (II)

R²-OH (II),

in der R¹ und R² unabhängig voneinander einen C₂-bis C₄-Alkylrest bedeuten, enthaltenden Gemischs, bei dem der Alkohol (II) einen um mindestens 1°C höheren Siedepunkt, gemessen bei oder extrapoliert auf 0,1 MPa abs, als der Vinylether (II) aufweist, gefunden, welches dadurch gekennzeichnet ist, dass man
a) das Gemisch in eine erste Destillationskolonne leitet und als Kopfprodukt ein Vinylether (1) und Alkohol (II) enthaltendes Azeotrop und als Sumpfprodukt einen mit dem Alkohol (II) angereicherten Strom entnimmt;
b) das Vinylether (I) und Alkohol (II) enthaltende Azeotrop aus der ersten Destillationskolonne in eine zweite Destillationskolonne, welche bei gegenüber der ersten Destillationskolonne um einen 0,01 bis 3 MPa höheren Druck betrieben wird, leitet und als Sumpfprodukt oder gasförmigen Seitenabzug im Abtriebsteil den Vinylether (I) und als Kopfprodukt ein Vinylether (I) und Alkohol (II) enthaltendes Azeotrop entnimmt; und
c) das Vinylether (I) und Alkohol (II) enthaltende Azeotrop aus der zweiten Destillationskolonne in die erste Destillationskolonne zurückführt.

Das erfindungsgemäße Verfahren umfasst somit zwei Destillationskolonnen. In der ersten Destillationskolonne wird das einzusetzende Vinylether (I) und Alkohol (II) enthaltende Gemisch in ein Vinylether (I) und Alkohol (II) enthaltendes Azeotrop als Kopfprodukt und einen mit dem Alkohol (II) angereicherten Strom als Sumpfprodukt getrennt.

Im Allgemeinen betreibt man die erste Destillationskolonne bei einer Temperatur von 75 bis 225°C und bevorzugt von 100 bis 175°C, gemessen im Sumpf der Kolonne, wobei die zu wählende Temperatur hauptsächlich abhängig ist vom zu trennenden Vinylether (I) und Alkohol (II) und dem gewählten Druck in der Destillationskolonne. Im Allgemeinen betreibt man die erste Destillationskolonne bei einem Druck von 0,01 bis 1 MPa abs und bevorzugt von 0,05 bis 0,5°MPa abs, gemessen am Kopf der Kolonne, wobei der zu wählende Druck hauptsächlich abhängig ist vom zu trennenden Vinylether (I) und Alkohol (II) und der gewählten Temperatur in der Destillationskolonne. Die jeweils für ein konkretes System zu wählende Temperatur und der zu wählende Druck kann vom Fachmann durch einfache Berechnung oder einfache Routineversuche ermittelt werden. Dabei existiert für jedes konkrete System ein Zusammenhang zwischen Temperatur und Druck, so dass der anzuwendende Parameterbereich in der Regel durch technische (beispielsweise Auslegung der Destillationskolonne), wirtschaftliche (beispielsweise Investitionskosten und/oder Energiekosten) und chemische (beispielsweise keine oder nur unwesentliche Zersetzung der Produkte) Randbedingungen festgelegt wird.

Im Allgemeinen weist die erste Destillationskolonne eine Anzahl theoretischer Trennstufen von 5 bis 75 auf, wobei die für ein konkretes System einzusetzende Anzahl der theoretischen Böden hauptsächlich abhängig ist vom zu trennenden Vinylether (I) und Alkohol (II) sowie von der erwarteten Trennleistung. Die jeweils für ein konkretes System zu wählende Anzahl der theoretischen Trennstufen kann vom Fachmann durch einfache Berechnung oder einfache Routineversuche ermittelt werden.

Als erste Destillationskolonne kann prinzipiell jede Destillationskolonne eingesetzt werden, welche die technischen Randbedingungen, insbesondere die gewünschte Trennleistung, die erforderliche Temperaturbeständigkeit, die erforderliche Druckbeständigkeit und die geforderte Materialbeständigkeit erfüllt. In der Regel handelt es sich um Kolonnen mit einem Metallmantel und trennwirksame sowie nichttrennwirksame Einbauten. Als trennwirksame Einbauten kommen beispielsweise Böden oder Packungen in Betracht.

Beim erfindungsgemäßen Verfahren ist im Allgemeinen vorteilhaft, die erste Destillationskolonne so auszulegen und zu betreiben, dass ≥ 50%, bevorzugt ≥ 75% und besonders bevorzugt ≥ 90% der mit dem Gemisch zugeführten Menge an Alkohol (II) über das Sumpfprodukt entnommen werden.

Die Konzentration des Alkohols (II) in dem als Sumpfprodukt zu entnehmenden Stroms beträgt im Allgemeinen ≥ 90 Gew.-% und bevorzugt ≥ 95 Gew.-%.

In der zweiten Destillationskolonne wird das Vinylether (I) und Alkohol (II) enthaltende Azeotrop aus der ersten Destillationskolonne in ein Vinylether (I) und Alkohol (II) enthaltendes Azeotrop als Kopfprodukt und dem Vinylether (I) als Sumpfprodukt oder als gasförmigen Seitenabzug im Abtriebsteil getrennt.

Im Allgemeinen betreibt man die zweite Destillationskolonne bei einer Temperatur von 75 bis 225°C und bevorzugt von 100 bis 175°C, gemessen im Sumpf der Kolonne, wobei die zu wählende Temperatur hauptsächlich abhängig ist vom zu trennenden Vinylether (I) und Alkohol (II) und dem gewählten Druck in der Destillationskolonne. Um eine entsprechende Trennung in ein Vinylether (I) und Alkohol (II) enthaltendes Azeotrop und einem Vinylether (I) Strom zu erreichen, betreibt man die zweite Destillationskolonne gegenüber der ersten Destillationskolonne bei einem um 0,01 bis 3 MPa höheren Druck. Bevorzugt betreibt man die zweite Destillationskolonne gegenüber der ersten Destillationskolonne bei einem um 0,1 bis 2 MPa höheren Druck. Ein wesentlicher Parameter bei der Festlegung des anzuwendenden Drucks ist hierbei die gewünschte Trennleistung. Dabei ist die Trennleistung in der zweiten Destillationskolonne in der Regel umso höher, je größer die Druckdifferenz zur ersten Destillationskolonne ist. Mit steigender Druckdifferenz zur ersten Destillationskolonne steigt auch der Anteil an Vinylether (I), welcher als Sumpfprodukt oder als gasförmiger Seitenabzug im Abtriebsteil entnommen werden kann. Das verbleibende Azeotrop ist somit bei hohem Druck ärmer an Vinylether (I), was letztendlich den Mengenstrom und die Belastung der ersten Destillationskolonne verringert. Demgegenüber ist zu beachten, dass mit steigender Druckdifferenz mehr Verdichtungsenergie benötigt wird, die zweite Destillationskolonne entsprechend für den entsprechenden Druck ausgelegt sein sollte und für ein konkretes, zu trennendes Gemisch auch die Destillationstemperatur steigt, was zu einer höheren thermischen Belastung der zu trennenden Produkte führt. Die jeweils für ein konkretes System zu wählende Temperatur und der zu wählende Druck kann vom Fachmann durch einfache Berechnung oder einfache Routineversuche ermittelt werden. Dabei existiert für jedes konkrete System ein Zusammenhang zwischen Temperatur und Druck, so dass der anzuwendende Parameterbereich in der Regel durch technische (beispielsweise Auslegung der Destillationskolonne, Trennleistung, Mengenströme), wirtschaftliche (beispielsweise Investitionskosten und/oder Energiekosten) und chemische (beispielsweise keine oder nur unwesentliche Zersetzung der Produkte) Randbedingungen festgelegt wird.

Im Allgemeinen weist die zweite Destillationskolonne eine Anzahl theoretischer Trennstufen von 5 bis 75 auf, wobei die für ein konkretes System einzusetzende Anzahl der theoretischen Trennstufen hauptsächlich abhängig ist vom zu trennenden Vinylether (I) und Alkohol (II) sowie von der erwarteten Trennleistung. Die jeweils für ein konkretes System zu wählende Anzahl der theoretischen Trennstufen kann vom Fachmann durch einfache Berechnung oder einfache Routineversuche ermittelt werden.

Als zweite Destillationskolonne kann prinzipiell jede Destillationskolonne eingesetzt werden, welche die technischen Randbedingungen, insbesondere die gewünschte Trennleistung, die erforderliche Temperaturbeständigkeit, die erforderliche Druckbeständigkeit und die geforderte Materialbeständigkeit erfüllt. In der Regel handelt es sich um Kolonnen mit einem Metallmantel und trennwirksame sowie nichttrennwirksame Einbauten. Als trennwirksame Einbauten kommen beispielsweise Böden oder Packungen in Betracht.

Bei der zweiten Destillationskolonne erfolgt die Entnahme des Vinylethers (I) flüssig als Sumpfprodukt oder gasförmig als Seitenabzug im Abtriebsteil. Im letztgenannten Fall erfolgt die Entnahme des Vinylethers (I) bevorzugt im Bereich der unteren 25% und besonders bevorzugt im Bereich der unteren 10% der Gesamtzahl der theoretischen Trennstufen. So bedeutet beispielsweise die Formulierung im Bereich der unteren 25% der Gesamtzahl der theoretischen Trennstufen bei einer Destillationskolonne mit insgesamt 75 theoretischen Trennstufen, dass der gasförmige Seitenabzug im Bereich des ersten bis 19. theoretischen Trennstufe entnommen wird. Der Vorteil des gasförmigen Seitenabzugs liegt in der Verbesserung der Farbzahl des rein zu gewinnenden Vinylethers (1), da unerwünschte Schwersieder aus dem Produkt ferngehalten werden können.

Beim erfindungsgemäßen Verfahren ist im Allgemeinen vorteilhaft, die zweite Destillationskolonne so auszulegen und zu betreiben, dass ≥ 20%, bevorzugt ≥ 50%, besonders bevorzugt ≥ 75% und ganz besonders bevorzugt ≥ 90% der mit dem Gemisch zugeführten Menge an Vinylether (I) über das Sumpfprodukt entnommen werden. Werte < 20% führen im Allgemeinen zu riesigen und somit unwirtschaftlichen Rückführungsströmen. Die Konzentration des Vinylethers (I) in dem als Sumpfprodukt zu entnehmenden Stroms beträgt im Allgemeinen ≥ 97,5 Gew.-% und bevorzugt ≥ 99,5 Gew.-%.

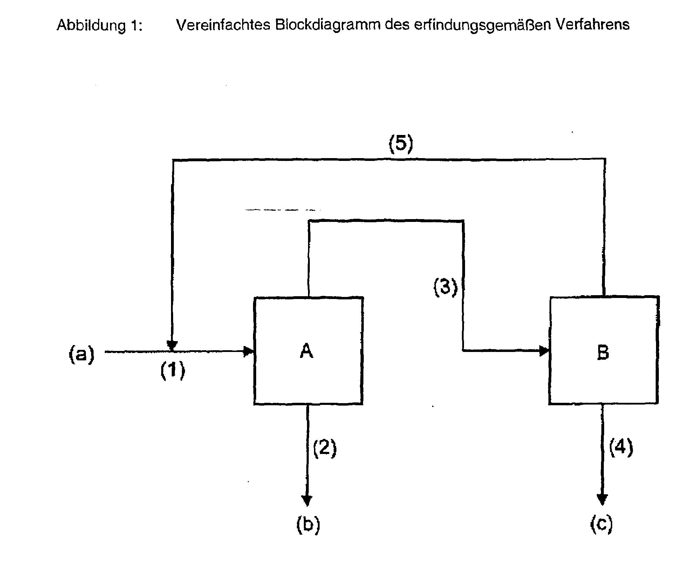

Abbildung 1 zeigt ein vereinfachtes Blockdiagramm des erfindungsgemäßen Verfahrens. Der zu trennende Vinylether (I) und Alkohol (II) enthaltende Strom (a) wird über Leitung (1) der ersten Destillationskolonne A zugeführt. Als Sumpfprodukt wird über Leitung (2) ein mit Alkohol (II) angereicherter Strom (b) entnommen. Das Vinylether (I) und Alkohol (II) enthaltende Azeotrop, welches als Kopfprodukt entnommen wird, wird über Leitung (3) der zweiten Destillationskolonne B zugeführt. Aus dieser wird als Sumpfprodukt oder als gasförmiger Seitenabzug im Abtriebsteil der Vinylether (I) (c) über Leitung (4) entnommen. Das Vinylether (I) und Alkohol (II) enthaltende Azeotrop, welches als Kopfprodukt entnommen wird, wird über Leitung (5) zur ersten Destillationskolonne A zurückgeführt.

Da der als Sumpfprodukt oder als gasförmiger Seitenabzug im Abtriebsteil der zweiten Destillationskolonne entnommene Vinylether (I) in der Regel noch mit geringfügigen Nebenkomponenten wie beispielsweise Acetalen verunreinigt ist, leitet man diesen bevorzugt in eine Reindestillationskolonne und gewinnt den den aufgereinigten Vinylether (I) als Kopfprodukt. Die Auslegung der Reindestillationskolonne und die Ermittlung der Destillationsparameter kann vom Fachmann durch einfache Berechnung oder einfache Routineversuche ermittelt werden.

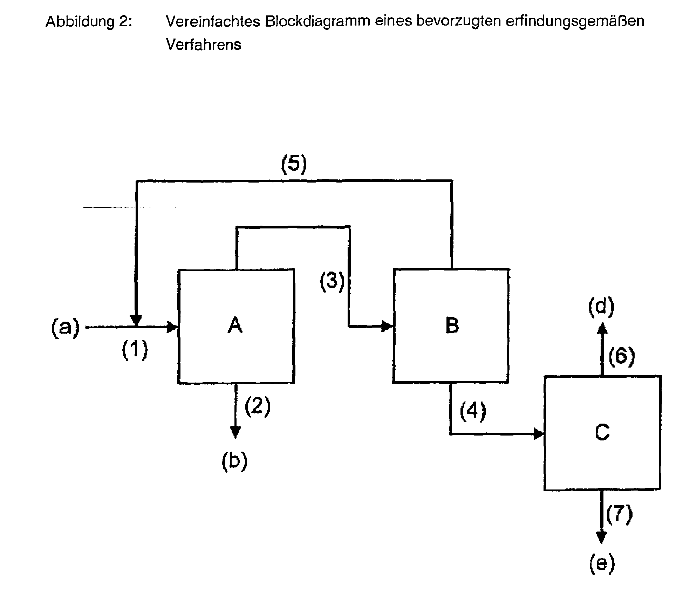

Abbildung 2 zeigt ein vereinfachtes Blockdiagramm diese bevorzugten erfindungsgemäßen Verfahrens. Der als Sumpfprodukt oder als gasförmiger Seitenabzug im Abtriebsteil der zweiten Destillationskolonne entnommene Vinylether (I) enthaltende Strom wird über Leitung (4) der Reindestillationskolonne C zurgeführt. Als Sumpfprodukt wird über Leitung (7) ein Strom mit den höhersiedenden Nebenkomponenten (e) entnommen. Der aufgereinigte Vinylether (I) (d) wird als Kopfprodukt über Leitung (6) entnommen.

Das beim erfindungsgemäßen Verfahren einzusetzende Gemisch enthält einen Vinylether (I) der allgemeinen Formel (I)

R¹-O-CH=CH₂ (I),

und einen Alkohol der allgemeinen Formel (II)

R²-OH (II),

in der die Reste R¹ und R² unabhängig voneinander einen C₂- bis C₄-Alkylrest bedeuten, enthält, und bei dem der Alkohol (II) einen um mindestens 1 °C höheren Siedepunkt, gemessen bei oder extrapoliert auf 0,1 MPa abs, als der Vinylether (II) aufweist.

Bevorzugt weist der Alkohol (II) einen um mindestens 2°C und besonders bevorzugt einen um mindestens 5°C höheren Siedepunkt, gemessen bei oder extrapoliert auf 0,1 MPa abs, als der Vinylether (II) auf.

Besonders bevorzugt setzt man beim erfindungsgemäßen Verfahren ein Vinylether (I) und Alkohol (II) enthaltendes Gemisch ein, bei dem die Reste R¹ und R² unabhängig voneinander einen C₂- bis C₄-Alkylrest, insbesondere Ethyl, 1-Propyl, 2-Propyl (sek. Propyl), 1-Butyl, 2-Butyl (sek. Butyl), 2-Methyl-1-propyl (iso-Butyl) und 2-Methyl-2-propyl (tert.-Butyl) bedeuten.

Ganz besonders bevorzugt setzt man beim erfindungsgemäßen Verfahren ein Vinylether (I) und Alkohol (II) enthaltendes Gemisch ein, bei dem die Reste R¹ und R² identisch sind. Ganz besonders bevorzugt ist somit der Einsatz folgender Vinylether (I) und Alkohol (II) enthaltender Gemische:
- Ethylvinylether und Ethanol;
- 1-Propylvinylether und 1-Propanol;
- 2-Propyvinylether und 2-Propanol;
- 1-Butylvinylether und 1-Butanol;
- 2-Butylvinylether und 2-Butanol;
- Isobutylvinylether und Isobuatnol;
- tert.-Butylvinylether und tert.-Butanol.

In einer bevorzugten Ausführungsform des erfindungsgemäßen Verfahrens stammt das eingesetzte Vinylether (I) und Alkohol (II) enthaltende Gemisch aus der Vinylether-Synthese durch Umsetzung des Alkohols (II) mit Ethin in Gegenwart einer basischen Alkali- oder Erdalkalimetallverbindung, trennt man aus dem mit dem Alkohol (II) angereicherten Sumpfprodukt aus der ersten Destillationskolonne Leichtsieder und Schwersieder destillativ ab und führt den aufgereinigten Alkohol (II) wieder zur Vinylether-Synthese zurück. Die Syntheseverfahren zur Herstellung von Vinylethern durch Umsetzung des Alkohols (II) mit Ethin in Gegenwart einer basischen Alkali- oder Erdalkalimetallverbindung sind allgemein bekannt und beispielsweise in Ullmann's Encyclopedia of Industrial Chemistry, 6th edition, 2000 Electronic Release, Chapter "VINYLETHERS - Production" oder W. Reppe et al., Justus Liebigs Ann. Chem., 601 (1956), Seiten 135 bis 138 und den darin zitierten Dokumenten beschrieben.

Die destillative Abtrennung der Leichtsieder und Schwersieder aus dem mit dem Alkohol (11) angereicherten Sumpfprodukt aus der ersten Destillationskolonne erfolgt beim zuvor genannten bevorzugten erfindungsgemäßen Verfahren in einer Trennwandkolonne oder einer Anordnung von konventionellen oder von thermisch und/oder stofflich gekoppelten Destillationskolonnen. Besonders bevorzugt erfolgt die genannte destillative Abtrennung in einer Trennwandkolonne oder einer Anordnung von thermisch und/oder stofflich gekoppelten Destillationskolonnen.

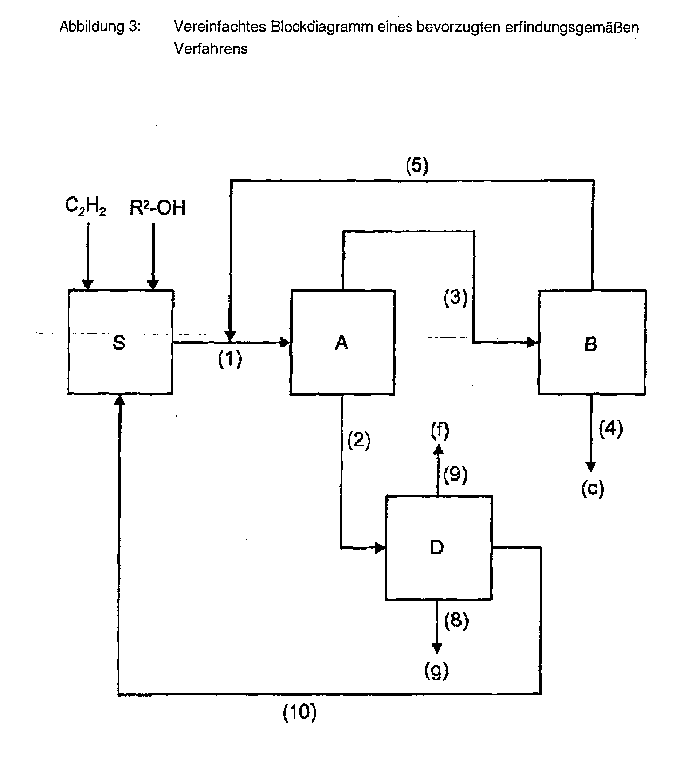

Abbildung 3 zeigt ein vereinfachtes Blockdiagramm dieses bevorzugten erfindungsgemäßen Verfahrens. Der als Sumpfprodukt der ersten Destillationskolonne entnommene Alkohol (II) enthaltende Strom wird über Leitung (2) zur Trennwandkolonne D beziehungsweise einer Anordnung von konventionellen oder thermisch und/oder stofflich gekoppelten Destillationskolonnen geführt. Dort erfolgt die Trennung in Leichtsieder (f), welche über Leitung (9) als Kopfprodukt und in Schwersieder (g), welche über Leitung (8) als Sumpfprodukt entnommen werden. Der aufgereinigte Alkohol (II) wird aus der Trennwandkolonne D beziehungsweise der Anordnung von konventionellen oder thermisch und/oder stofflich gekoppelten Destillationskolonnen als Seitenabzug entnommen und über Leitung (10) zur Synthesstufe S zurückgeführt.

In einer bevorzugten Ausführungsform des erfindungsgemäßen Verfahrens setzt man ein Vinylether (I) und Alkohol (II) enthaltendes Gemisch ein, welches aus der Vinylether-Synthese durch Umsetzung des Alkohols (II) mit Ethin in Gegenwart einer basischen Alkali- oder Erdalkalimetallverbindung stemmt und bei dem die Rest R¹ und R² identisch sind und für einen C₂- bis C₄-Alkylrest stehen. Dieses Gemisch wird analog der Beschreibung zum vereinfachten Blockdiagramm der Abbildung 1 in einen Alkohol (II) enthaltenden Strom und einen Vinylether (I) enthaltenden Strom getrennt. Falls aufgrund der erzielten und gewünschten Reinheit der den Alkohol (II) enthaltende Strom weiter aufzureinigen ist, wird dieser bevorzugt analog der Beschreibung zum vereinfachten Blockdiagramm der Abbildung 3 durch eine nachfolgende Destillation in einer Trennwandkolonne oder einer Anordnung von thermisch und/oder stofflich gekoppelten Destillationskolonnen aufgreinigt. Der erhaltene Alkohol (II) enthaltende Strom wird anschließend zur Synthesstufe zurückgeführt. Der Vinylether (I) enthaltende Strom wird analog der Beschreibung zum vereinfachten Blockdiagramm der Abbildung 2 durch eine nachfolgende Destillation aufgereinigt und der aufgereinigte Vinylether (I) als Kopfprodukt gewonnen.

Bei der genannten bevorzugten Ausführungsform umfasst die erste Destillationskolonne im Allgemeinen 5 bis 75 theoretische Trennstufen und wird bei einem Druck von 0,01 bis 1 MPa abs, gemessen am Kopf der Kolonne, und einer Temperatur von 75 bis 225°C, gemessen im Sumpf der Kolonne, betrieben. Die zweite Destillationskolonne umfasst im Allgemeinen 5 bis 75 theoretische Trennstufen und wird bei einem um 0,1 bis 2 MPa höheren Druck als die erste Destillationskolonne und einer Temperatur von 75 bis 225°C, gemessen im Sumpf der Kolonne, betrieben. Wird der Vinylether (1) aus der zweiten Destillationskolonne als gasförmiger Seitenabzug im Abtriebsteil entnommen, so befindet sich dieser Seitenabzug im Allgemeinen im Bereich des ersten bis zehnten und bevorzugt im Bereich des ersten bis zweiten theoretischen Bodens.

Das erfindungsgemäße Verfahren ermöglicht die Trennung eines Vinylether und Alkohol enthaltenden Gemischs, welches insbesondere mit geringem apparativen und verfahrenstechnischen Aufwand, mit hoher Anlagen-Kapazität und ohne die Gefahr einer Verunreinigung des Vinylethers und/oder des Alkohols durch die Zugabe von Fremdsubstanzen als Hilfsmittel zu aufgereinigtem Vinylether und Alkohol führt.

## Patentansprüche

1. Verfahren zur destillativen Trennung eines Vinylether der allgemeinen Formel (I)
R¹-O-CH=CH₂ (I),
und Alkohol der allgemeinen Formel (II)
R²-OH (II),
in der R¹ und R² unabhängig voneinander einen C₂- bis C₄-Alkylrest bedeuten, enthaltenden Gemischs, bei dem der Alkohol (II) einen um mindestens 1°C höheren Siedepunkt, gemessen bei oder extrapoliert auf 0,1 MPa abs, als der Vinylether (II) aufweist, **dadurch gekennzeichnet, dass** man
a) das Gemisch in eine erste Destillationskolonne leitet und als Kopfprodukt ein Vinylether (I) und Alkohol (II) enthaltendes Azeotrop und als Sumpfprodukt einen mit dem Alkohol (II) angereicherten Strom entnimmt;
b) das Vinylether (I) und Alkohol (II) enthaltende Azeotrop aus der ersten Destillationskolonne in eine zweite Destillationskolonne, welche bei gegenüber der ersten Destillationskolonne um einen 0,01 bis 3 MPa höheren Druck betrieben wird, leitet und als Sumpfprodukt oder gasförmigen Seitenabzug im Abtriebsteil den Vinylether (I) und als Kopfprodukt ein Vinylether (I) und Alkohol (II) enthaltendes Azeotrop entnimmt; und
c) das Vinylether (I) und Alkohol (II) enthaltende Azeotrop aus der zweiten Destillationskolonne in die erste Destillationskolonne zurückführt.

2. Verfahren nach Anspruch 1, **dadurch gekennzeichnet, dass** man die zweite Destillationskolonne um einen um 0,1 bis 2 MPa höheren Druck, gemessen am Kopf der Kolonne, betreibt als die erste Destillationskolonne.

3. Verfahren nach den Ansprüchen 1 bis 2, **dadurch gekennzeichnet, dass** man die erste Destillationskolonne bei einer Temperatur von 75 bis 225°C, gemessen im Sumpf der Kolonne und einem Druck von 0,01 bis 1 MPa abs, gemessen am Kopf der Kolonne, betreibt.

4. Verfahren nach den Ansprüchen 1 bis 3, **dadurch gekennzeichnet, dass** man die zweite Destillationskolonne bei einer Temperatur von 75 bis 225°C, gemessen im Sumpf der Kolonne, betreibt.

5. Verfahren nach den Ansprüchen 1 bis 4, **dadurch gekennzeichnet, dass** man in der zweiten Destillationskolonne den Vinylether (I) als gasförmigen Seitenabzug im Abtriebsteil im Bereich der unteren 25% der Gesamtzahl der theoretischen Trennstufen entnimmt:

6. Verfahren nach den Ansprüchen 1 bis 5, **dadurch gekennzeichnet, dass** man den aus der zweiten Destillationskolonne als Sumpfprodukt oder gasförmigen Seitenabzug im Abtriebsteil entnommenen Vinylether (I) in eine Reindestillationskolonne leitet und daraus den aufgereinigten Vinylether (I) als Kopfprodukt gewinnt.

7. Verfahren nach den Ansprüchen 1 bis 6, **dadurch gekennzeichnet, dass** man ein Vinylether (I) und Alkohol (II) enthaltendes Gemisch einsetzt, bei dem die Reste R¹ und R² identisch sind.

8. Verfahren nach Anspruch 7, **dadurch gekennzeichnet, dass** das eingesetzte Vinylether (I) und Alkohol (II) enthaltende Gemisch aus der Vinylether-Synthese durch Umsetzung des Alkohols (II) mit Ethin in Gegenwart einer basischen Alkali- oder Erdalkalimetallverbindung stammt, man aus dem mit dem Alkohol (II) angereicherten Sumpfprodukt aus der ersten Destillationskolonne Leichtsieder und Schwersieder destillativ abtrennt und man den aufgereinigten Alkohol (II) wieder zur Vinylether-Synthese zurückführt.

9. Verfahren nach Anspruch 8, **dadurch gekennzeichnet, dass** man die destillative Abtrennung der Leichtsieder und Schwersieder aus dem mit dem Alkohol (II) angereicherten Sumpfprodukt aus der ersten Destillationskolonne in einer Trennwandkolonne oder einer Anordnung von thermisch und/oder stofflich gekoppelten Destillationskolonnen durchführt.

## Claims

1. A process for distillatively separating a mixture comprising a vinyl ether of the general formula (I)
R¹-O-CH=CH₂ (I)
and alcohol of the general formula (II)
R²-OH (II)
in which R¹ and R² are each independently a C₂-C₄-alkyl radical, and in which the alcohol (II) has a boiling point which is at least 1°C higher, measured at or extrapolated to 0.1 MPa abs, than the vinyl ether (I), which comprises
a) passing the mixture into a first distillation column and withdrawing, as a top product, an azeotrope comprising vinyl ether (I) and alcohol (II) and, as a bottom product, a stream enriched with the alcohol (II);
b) passing the azeotrope comprising vinyl ether (I) and alcohol (II) from the first distillation column into a second distillation column which is operated at a pressure which is from 0.01 to 3 MPa higher compared to the first distillation column, and withdrawing, as a bottom product or gaseous sidestream in the stripping section, the vinyl ether (I) and, as a top product, an azeotrope comprising vinyl ether (I) and alcohol (II); and
c) recycling the azeotrope comprising vinyl ether (I) and alcohol (II) from the second distillation column into the first distillation column.

2. The process according to claim 1, wherein the second distillation column is operated at a pressure, measured at the top of the column, which is from 0.1 to 2 MPa higher than the first distillation column.

3. The process according to either of claims 1 to 2, wherein the first distillation column is operated at a temperature of from 75 to 225°C, measured in the bottom of the column, and a pressure of from 0.01 to 1 MPa abs, measured at the top of the column.

4. The process according to any of claims 1 to 3, wherein the second distillation column is operated at a temperature of from 75 to 225°C, measured in the bottom of the column.

5. The process according to any of claims 1 to 4, wherein the vinyl ether (I) is withdrawn as a gaseous sidestream in the stripping section of the second distillation column in the region of the lower 25% of the total number of theoretical plates.

6. The process according to any of claims 1 to 5, wherein the vinyl ether (I) withdrawn from the second distillation column as a bottom product or gaseous sidestream in the stripping section is passed into a purifying distillation column and the purified vinyl ether (I) is obtained therefrom as a top product.

7. The process according to any of claims 1 to 6, wherein a mixture comprising vinyl ether (I) and alcohol (II) is used in which the R¹ and R² radicals are identical.

8. The process according to claim 7, wherein the mixture used which comprises vinyl ether (I) and alcohol (II) stems from the vinyl ether synthesis by reacting the alcohol (II) with ethyne in the presence of a basic alkali metal or alkaline earth metal compound, distillatively removing low boilers and high boilers from the bottom product enriched with the alcohol (II) in the first distillation column and recycling the purified alcohol (II) back to the vinyl ether synthesis.

9. The process according to claim 8, wherein the distillative removal of low boilers and high boilers from the bottom product enriched with the alcohol (II) in the first distillation column is carried out in a dividing wall column or an arrangement of distillation columns having heat and/or mass transfer.

## Revendications

1. Procédé de séparation par distillation d'un mélange contenant du vinyléther de formule générale (I) :
**R¹-O-CH=CH₂** **(I),**
et un alcool de formule générale (II) :
**R²-OH** **(II),**
dans lesquelles R¹ et R² représentent, indépendamment l'un de l'autre, un radical alkyle en C₂-C₄, procédé dans lequel l'alcool (II) présente un point d'ébullition, mesuré ou extrapolé à une pression absolue de 0,1 MPa, supérieur d'au moins 1 °C à celui du vinyléther (I), **caractérisé en ce que** :
a) on achemine le mélange dans une première colonne de distillation et on prélève comme produit de tête un azéotrope contenant le vinyléther (I) et l'alcool (II) et comme produit de bas de colonne un courant enrichi en alcool (II) ;
b) on achemine l'azéotrope contenant le vinyléther (I) et l'alcool (II) de la première colonne de distillation dans une seconde colonne de distillation, qui est activée à une pression plus élevée de 0,01 à 3 MPa par rapport à celle de la première colonne de distillation, et on prélève le vinyléther (I) comme produit de bas de colonne ou comme soutirage latéral gazeux dans la partie de stripage, et un azéotrope contenant le vinyléther (I) et l'alcool (II), comme produit de tête; et
c) on renvoie l'azéotrope contenant le vinyléther (I) et l'alcool (II) de la seconde colonne de distillation dans la première colonne de distillation.

2. Procédé selon la revendication 1, **caractérisé en ce que** l'on fait fonctionner la seconde colonne de distillation à une pression, mesurée en tête de colonne, supérieure de 0,1 à 2 MPa à celle de la première colonne de distillation.

3. Procédé selon les revendications 1 et 2, **caractérisé en ce que** l'on fait fonctionner la première colonne de distillation à une température de 75 à 225 °C, mesurée en bas de colonne, et à une pression absolue de 0,01 à 1 MPa, mesurée en tête de colonne.

4. Procédé selon les revendications 1 à 3, **caractérisé en ce que** l'on fait fonctionner la seconde colonne de distillation à une température de 75 à 225 °C, mesurée en bas de colonne.

5. Procédé selon les revendications 1 à 4, **caractérisé en ce que**, dans la seconde colonne de distillation, on retire le vinyléther (I) comme soutirage latéral gazeux, dans la partie de stripage, dans la plage des 25 % inférieurs du nombre total d'étapes de séparation théoriques.

6. Procédé selon les revendications 1 à 5, **caractérisé en ce que** l'on achemine le vinyléther (I) retiré de la seconde colonne de distillation comme produit de bas de colonne ou comme soutirage latéral gazeux dans la partie de stripage, dans une colonne de pure distillation et **en ce que** l'on obtient de la sorte le vinyléther (I) purifié comme produit de tête.

7. Procédé selon les revendications 1 à 6, **caractérisé en ce que** l'on utilise un mélange contenant du vinyléther (I) et de l'alcool (II), dans lequel les radicaux R¹ et R² sont identiques.

8. Procédé selon la revendication 7, **caractérisé en ce que** le mélange utilisé contenant du vinyléther (I) et de l'alcool (II) provient de la synthèse de vinyléther par réaction de l'alcool (II) avec de l'éthine en présence d'un composé de métal alcalin ou de métal alcalinoterreux basique, **en ce que** l'on sépare par distillation les substances à bas points d'ébullition et les substances à hauts points d'ébullition du produit de bas de colonne enrichi en alcool (II) issu de la première colonne de distillation et **en ce que** l'on renvoie l'alcool purifié (II) à la synthèse de vinyléther.

9. Procédé selon la revendication 8, **caractérisé en ce que** l'on réalise la séparation par distillation des substances à bas points d'ébullition et des substances à hauts points d'ébullition du produit de bas de colonne enrichi en alcool (II) issu de la première colonne de distillation dans une colonne à paroi séparatrice ou dans un dispositif de colonnes de distillation couplées thermiquement et/ou matériellement.
